# FASCICULE DE BREVET EUROPEEN

(11) **EP 1 068 865 B1**
(45) Date de publication et mention de la délivrance du brevet: **16.03.2005**
(21) Numéro de dépôt: 00401743.0
(22) Date de dépôt: 19.06.2000
(51) Int. Cl.: A61K 7/48, A61K 7/06

(54) **Utilisation de l'acide 4,6-diméthoxy-indole 2-carboxylique ou de ses dérivés pour le traitement de la séborrhée**
Verwendung von 4,6-Dimethoxy-Indol 2-Carbonsäure oder Derivaten davon zur Seborrhea Behandlung
Use of 4,6-dimethoxy-indole 2-carboxylic acid or derivatives thereof for seborrhea treatment

(30) Priorité: 16.07.1999 FR 9909269
(43) Date de publication de la demande: 17.01.2001
(73) Titulaire: L'OREAL, 75008 Paris (FR)
(72) Inventeur: Dalko, Maria, 91190 Gif-sur-Yvette (FR); Galey, Jean-Baptiste, 93600 Aulnay-Sous-Bois (FR); Bernard, Bruno, 92200 Neuilly-sur-Seine (FR)
(74) Mandataire: Galup, Cédric Olivier Nicolas

(56) Documents cités:
- WO-A-99/07351
- WO-A-99/12905

## Description

L'invention concerne l'utilisation dans une composition ou pour la préparation d'une composition d'une quantité efficace d'acide 4,6-diméthoxy-indole 2-carboxylique ou de ses dérives, le composé ou la composition étant destinés à traiter la séborrhée et les dermatoses qui lui sont associées, notamment l'acné et/ou les points noirs et/ou les comédons.

La séborrhée ou hypersécrétion sébacée est à l'origine des troubles cutanés que sont la peau grasse et le cuir chevelu gras, désordres esthétiques qui peuvent devenir le lit d'affections dermatologiques comme l'acné, la dermite séborrhéique, les points noirs ou encore les comédons.

L'acné est l'une des maladies affectant le plus souvent, et à des degrés divers, la population juvénile entre 15 et 30 ans. L'acné est essentiellement la conséquence de deux phénomènes intriqués : l'hypersécrétion sébacée et la perturbation de la kératinisation du follicule pilosébacé, dont les conséquences sont l'obstruction du follicule pilosébacé et la formation de lésions rétentionnelles ou comédons. Les comédons, par suite de la prolifération microbienne, peuvent évoluer en lésions inflammatoires, papules et pustules.

La dermite séborrhéique est associée à la prolifération de levures du genre *Pityrosporum* sur le substrat que constitue pour elles le sébum.

Dans le cas de l'acné comme dans celui de la dermite séborrhéique, la régulation du flux sébacé supprime la cause initiale, c'est-à-dire la présence excessive de sébum, et traite par voie de conséquence la dermatose.

C'est ce que l'on observe avec l'isotrétinoïne dont l'administration par voie orale induit un assèchement des follicules sébacés et conduit à la disparition des symptômes.

Mais l'utilisation de l'isotrétinoïne n'est pas dénuée d'effets secondaires sérieux et reste de ce fait réservée au traitement des acnés sévères, invalidantes. Les traitements de la séborrhée par voie topique se sont avérés jusqu'à présent peu efficaces et on pallie fréquemment ce manque d'efficacité par des traitements par voie systémique, notamment avec l'isotrétinoïne ou des anti-androgènes.

On constate donc que subsiste le besoin d'actifs topiques ayant un effet sur l'hypersécrétion sébacée et par voie de conséquence sur les dermatoses qui lui sont associées.

C'est ce qu'apporte la présente invention.

La demanderesse a de manière surprenante et inattendue découvert que l'acide 4,6-diméthoxy-indole 2-carboxylique ou ses dérivés présentent des propriétés anti-séborrhéiques et peuvent donc être utilisés dans une composition en tant qu'actif pour lutter contre la séborrhée de la peau ou du cuir chevelu, et contre les dermatoses qui peuvent en découler comme l'acné, la dermite séborrhéique, les points noirs et/ou les comédons.

Ainsi, la présente invention a pour objet l'utilisation dans une composition ou pour la préparation d'une composition, d'une quantité efficace d'au moins un composé répondant à la formule générale (I): 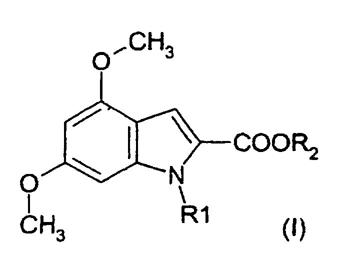 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls ou en mélange en toutes proportions ;
ledit composé et/ou lesdites compositions étant destinés à traiter la séborrhée de la peau et/ou du cuir chevelu, et/ou les dermatoses associées à la séborrhée.

La dermatose associée à la séborrhée peut notamment être l'acné et/ou les points noirs et/ou les comédons.

Ces composés présentent des activités remarquables qui justifient leur utilisation comme principe actif pour traiter la séborrhée de la peau et/ou du cuir chevelu, et les dermatoses associées à la séborrhée comme l'acné et/ou les points noirs et/ou les comédons, et ceci à titre préventif et/ou curatif.

A la connaissance de la demanderesse, il n'a jamais été proposé dans l'art antérieur l'utilisation de tels composés pour traiter la séborrhée de la peau et/ou du cuir chevelu, et les dermatoses associées à la séborrhée comme l'acné et/ou les points noirs et/ou les comédons.

Par hétérocycle, on entend de préférence selon l'invention un cycle incluant éventuellement un ou plusieurs atomes d'azote et/ou d'oxygène et particulièrement la pyridine, l'imidazole, le tétrahydrofuranne ou le furanne. Un hétérocycle particulièrement préféré selon l'invention est la pyridine.

Par radical alkyle en C₁-C₄ on entend selon invention les radicaux acycliques provenant de l'enlèvement d'un atome d'hydrogène dans la molécule d'un hydrocarbure, linéaires ou ramifiés ayant de 1 à 4 atomes de carbone et en particulier les radicaux méthyle, éthyle, propyle, isopropyle, butyle, isobutyle ou tertiobutyle.

Par radical aralkyle en C₇ - C₁₂, on entend de préférence selon l'invention les radicaux alkyles-aryles ayant de 7 à 12 atomes de carbone, définition dans laquelle le terme aryle s'entend comme un cycle aromatique ayant 5 ou 6 atomes de carbone ou un hétérocycle aromatique ayant 5 ou 6 atomes. Préférentiellement selon l'invention, le radical aralkyle est en C₇-C₁₀. Un radical aralkyle particulièrement préféré selon l'invention est le radical benzyle.

Par un radical phényle éventuellement substitué, on entend de préférence selon l'invention le radical phényle éventuellement substitué par un groupement cyano (-CN), un groupement trifluorométhyle (-CF₃), un radical méthoxy (-O-CH₃) ou un atome d'halogène. L'atome d'halogène peut être choisi parmi le chlore, le brome, le fluor, l'iode. Un radical phényle substitué particulièrement préféré selon l'invention est le radical phényle substitué par un groupement trifluorométhyle (-CF₃).

Selon une forme de réalisation préférée de l'invention R₁ représente un atome d'hydrogène ou un radical méthyle ou bien éthyle.

Selon une autre forme de réalisation préférée de l'invention R₂ représente un atome d'hydrogène ou un radical méthyle.

Selon une forme de réalisation très préférentielle de l'invention, R₁ et R₂ sont un atome d'hydrogène.

On peut citer comme composés de formule (I):
l'acide 4,6-diméthoxy-indole 2-carboxylique
l'acide 4,6-diméthoxy-indole 2-carboxylate de méthyle
l'acide N-méthyl-4,6-diméthoxy-indole 2-carboxylique
l'acide N-méthyl-4,6-diméthoxy-indole 2-carboxylate de méthyle
l'acide N-éthyl-4,6-diméthoxy-indole 2-carboxylique.

Parmi ces composés, on préfère tout particulièrement l'acide 4,6-diméthoxy-indole 2-carboxylique

Selon l'invention, les composés peuvent être utilisés seuls ou en mélange.

La quantité efficace de composé à utiliser correspond bien entendu à la quantité nécessaire pour obtenir le résultat désiré. L'homme du métier est donc en mesure d'évaluer cette quantité efficace qui dépend de la nature du composé utilisé et de la personne ainsi traitée.
Pour donner un ordre de grandeur, dans les compositions selon l'invention le composé représente présent à une concentration comprise entre 0,001% et 20% en poids par rapport au poids total de la composition et de préférence comprise entre 0,1% et 5%.

Selon l'invention, les composés de formule (I) peuvent être utilisés dans des compositions à usage cosmétique ou pharmaceutique. Préférentiellement selon l'invention, les composés de formule (I) sont utilisés dans des compositions à usage cosmétique

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles bien connues de l'homme de l'art du domaine.

Les compositions de l'invention peuvent comprendre les adjuvants classiquement mis en oeuvre dans les domaines considérés comme les corps gras, les solvants organiques, les épaississants, les adoucissants, les antioxydants, les opacifiants, les stabilisants, les agents anti-mousse, les parfums, les émulsionnants ioniques ou non, les charges, les séquestrants, les colorants ou tout autre ingrédient habituellement utilisé en cosmétique.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme huiles utilisables dans l'invention, on peut citer les huiles minérales (huile de vaseline), les huiles végétales (fraction liquide de beurre de karité), les huiles animales, les huiles de synthèse (huile de purcellin, polyisobutène hydrogénée), les huiles siliconées et les huiles fluorées (perfluoropolyéthers). On peut aussi utiliser comme matières grasses des alcools gras, des acides gras, des cires.

Comme émulsionnants utilisables dans l'invention, on peut citer par exemple les esters d'acide gras et de polyol, tels que les esters gras de sorbitol, comme le tristéarate de sorbitan vendu sous la dénomination de Span 65 par la société ICI, ou aussi les esters gras de glycérol tels que le monostéarate de glycérol, ou encore les esters de PEG tels que le stéarate de PEG-40 vendu sous la dénomination de Myrj 52 par la société ICI. Il peut s'agir aussi d'émulsionnants siliconés tels que le cétyl diméthicone copolyol vendu sous la dénomination d'Abil EM90 par la société Goldschmidt.

Comme gélifiants hydrophiles, on peut citer les polymères carboxyvinyliques (carbomer), les copolymères acryliques tels que les copolymères d'acrylates/alkylacrylates, les poly(méth)acrylates de glycéryle tels que le produit vendu sous la dénomination de Norgel par la société Guardian, les polyacrylamides et notamment le mélange de polyacrylamide, C13-14-lsoparaffine et Laureth-7, vendu sous la dénomination de Sepigel 305 par la société Seppic, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Les compositions de l'invention peuvent comprendre aussi des actifs, hydrophiles ou lipophiles, et notamment les actifs susceptibles de compléter l'effet de l'acide ascorbique dans le traitement de la séborrhée et des dermatoses associées, et notamment de l'acné. Il peut s'agir par exemple d'agents anti-inflammatoires tels que le peroxyde de benzoyle, d'antibiotiques, d'agents antiseptiques tels que l'octopirox ou d'actifs kératolytiques tels que l'acide salicylique et ses dérivés, les alpha-hydroxyacides, l'acide rétinoïque et ses dérivés, le rétinol et ses dérivés.

Par ailleurs, comme actifs hydrophiles, on peut utiliser par exemple les protéines ou les hydrolysats de protéine, les acides aminés, les polyols (glycérine, propylène glycol), l'urée, l'allantoïne, les sucres et les dérivés de sucre, les vitamines hydrosolubles, l'amidon, les extraits bactériens ou végétaux, notamment d'Aloe Vera, les agents hydratants.

Comme actifs lipophiles, on peut utiliser par exemple le tocophérol (vitamine E) et ses dérivés, les acides gras essentiels, les céramides, les huiles essentielles.

La présente invention a aussi pour objet un procédé de traitement cosmétique de la séborrhée et des dermatoses associées à la séborrhée, comme par exemple l'acné et/ou les points noirs et/ou les comédons, consistant à appliquer sur la peau et/ou le cuir chevelu, une composition comprenant une quantité efficace d'au moins un composé répondant à la formule générale (I), à laisser celle-ci en contact avec la peau et/ou le cuir chevelu, et éventuellement à rincer.

Préférentiellement, le procédé de traitement cosmétique de l'invention vise à traiter l'acné et/ou les points noirs et/ou les comédons.

Le procédé de traitement présente les caractéristiques d'un procédé cosmétique dans la mesure où il permet d'améliorer l'esthétique de la peau en lui donnant un aspect amélioré.

On va maintenant donner à titre d'illustration des exemples qui ne sauraient limiter en aucune façon la portée de l'invention.

### Exemples de compositions selon l'invention :

Ces compositions sont obtenues par les techniques habituelles couramment utilisées en cosmétique ou en pharmacie.

### Gel niosomé :

- Chimexane NS® 1,80 g
- Stéaroylglutamate monosodique 0,20 g
- acide 4,6-diméthoxy-indole-2-carboxylique 1,00 g
- Carbomer 0,20 g
- Triéthanolamine qspH = 7
- Conservateurs qs
- Parfums qs
- Eau déminéralisée qsp 100,00 g

On applique ce gel sur la peau, une à deux fois par jour.

### Lotion niosomée :

- Chimexane NL® 0,475 g
- Cholestérol 0,475 g
- Stéaroylglutamate monosodique 0,050 g
- acide 4,6-diméthoxy-indole-2-carboxylique 2,000 g
- Conservateurs qs
- Colorant s qs
- Parfum qs
- Eau déminéralisée qsp 100,000 g

On applique cette lotion sur la peau, une à deux fois par jour.

### lotion:

- acide 4,6-diméthoxy-indole-2-carboxylique 2,00 g
- Dowanol PM®** 20,00 g
- Klucel G®* 3,00 g
- Alcool éthylique 40,00 g
- Eau qsp 100,00 g

** : Monométhyléther de propylèneglycol vendu par la société Dow Chemical
* : Hydroxypropylcellulose vendue par la société Hercules

On applique 1 ml de cette lotion sur la peau, à la fréquence de une à deux fois par jour.

## Revendications

1. Utilisation cosmétique non thérapeutique dans une composition d'une quantité efficace d'au moins un composé répondant à la formule générale (I): 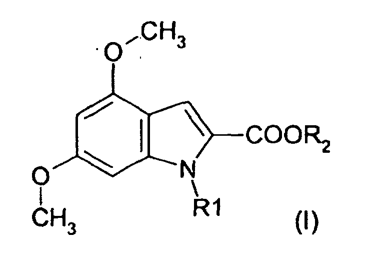 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls ou en mélange en toutes proportions ;
en tant qu'actif pour lutter contre la peau grasse et/ou le cuir chevelu gras.

2. Utilisation d'une quantité efficace d'au moins un composé répondant à la formule générale (I) : 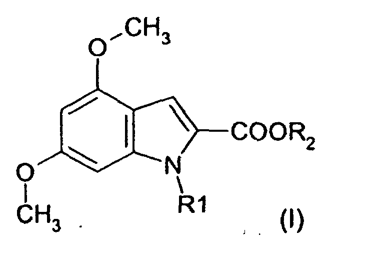 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂ ,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls ou en mélange en toutes proportions ;
pour la fabrication d'une composition destinée à traiter les dermatoses associées à la séborrhée.

3. Utilisation d'une quantité efficace d'au moins un composé répondant à la formule générale (I) : 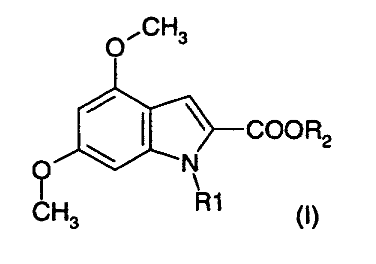 dans laquelle
R₁ et R₂, identiques ou différents, représentent :
un atome d'hydrogène,
ou un radical alkyle en C₁-C₆, éventuellement substitué par un radical -OH, -NHR₃, -SH, -COOH ou -COOR₃, dans lesquels R₃ représente un radical alkyle en C₁-C₄, linéaire ou ramifié,
ou un radical aralkyle en C₇ - C₁₂ ,
ou un radical -CHR₄R₅, dans lequel R₄ et R₅ identiques ou différents représentent un atome d'hydrogène ou un radical phényle éventuellement substitué ou encore un hétérocycle ayant 5 ou 6 chaînons ;
ses formes acylées ou encore ses sels physiologiquement acceptables pris seuls
ou en mélange en toutes proportions ;
pour la préparation d'une composition destinée à traiter la dermite séborrhéique et/ou l'acné et/ou les points noirs et/ou les comédons.

4. Utilisation selon l'une quelconque des revendications précédentes, **caractérisée par le fait que** R₁ est un atome d'hydrogène ou un radical méthyle ou bien éthyle.

5. Utilisation selon la revendication précédente, **caractérisée par le fait que** R₁ est un atome d'hydrogène.

6. Utilisation selon l'une quelconque des revendications précédentes, **caractérisée par le fait que** R₂ est un atome d'hydrogène ou un radical méthyle.

7. Utilisation selon la revendication précédente, **caractérisée par le fait que** R₂ est un atome d'hydrogène.

8. Utilisation selon l'une quelconque des revendications précédentes, **caractérisée par le fait que** le composé est choisi parmi
l'acide 4,6-diméthoxy-indole 2-carboxylique
l'acide 4,6-diméthoxy-indole 2-carboxylate de méthyle
l'acide N-méthyl-4,6-diméthoxy-indole 2-carboxylique
l'acide N-méthyl-4,6-diméthoxy-indole 2-carboxylate de méthyle
l'acide N-éthyl-4,6-diméthoxy-indole 2-carboxylique.

9. Utilisation selon la revendication précédente, **caractérisée par le fait que** le composé est l'acide 4,6-diméthoxy-indole 2-carboxylique.

10. Utilisation selon l'une quelconque des revendications précédentes, **caractérisée par le fait que** le composé présent est utilisé à une concentration comprise entre 0,001% et 20%, en poids par rapport au poids total de la composition.

11. Utilisation selon l'une quelconque des revendications précédentes, **caractérisée par le fait que** le composé présent est utilisé à une concentration comprise entre 0,1% et 5%, en poids par rapport au poids total de la composition.

12. Procédé de traitement cosmétique de la peau grasse et/ou du cuir chevelu gras, consistant à appliquer sur la peau et/ou le cuir chevelu, une composition comprenant une quantité efficace d'au moins un composé répondant à la formule générale (I) tel que définie dans l'une quelconque des revendications 1 à 11, à laisser celle-ci en contact avec la peau et/ou le cuir chevelu, et éventuellement à rincer.

## Claims

1. Non-therapeutic: cosmetic use, in a composition, of an effective amount of at least one compound corresponding to the general formula (I) : 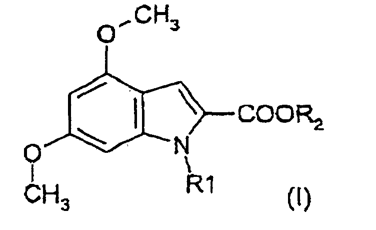 in which
R₁ and R₂, which may be identical or different, represent:
a hydrogen atom,
or a C₁-C₆ alkyl radical, optionally substituted with an -OH, -NHR₃, -SH, -COOH or -COOR₃ radical, in which R₃ represents a linear or branched C₁-C₄ alkyl radical,
or a C₇-C₁₂ aralkyl radical,
or a radical -CHR₄R₅ in which R₄ and R₅, which may be identical or different, represent a hydrogen atom or an optionally substituted phenyl radical or alternatively a 5- or 6-membered heterocycle;
the acylated forms thereof or alternatively the physiologically acceptable salts thereof, taken alone or as a mixture in any proportion;
as active principle for combating greasy skin and/or a greasy scalp.

2. Use of an effective amount of at least one compound corresponding to the general formula (I): 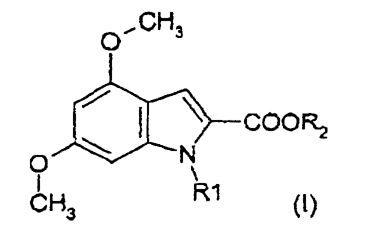 in which
R₁ and R₂, which may be identical or different, represent:
a hydrogen atom,
or a C₁-C₆ alkyl radical, optionally substituted with an -OH,- -NHR₃, -SH, -COOH or -COOR₃ radical, in which R₃ represents a linear or branched C₁-C₄ alkyl radical,
or a C₁-C₁₂ aralkyl radical,
or a radical -CHR₄R₅ in which R₄ and R₅, which may be identical or different, represent a hydrogen atom or an optionally substituted phenyl radical or alternatively a 5- or 6-membered heterocycle;
the acylated forms thereof or alternatively the physiologically acceptable salts thereof, taken alone or as a mixture in any proportion;
for the manufacture of a composition intended to treat the dermatitis associated with seborrhoea.

3. Use of an effective amount of at least one compound corresponding to the general formula (I) : 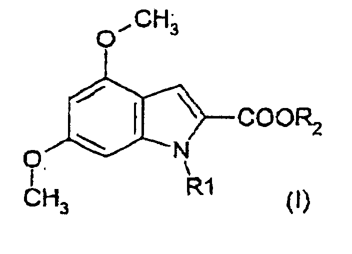 in which
R₁ and R₂, which may be identical or different, represent:
a hydrogen atom,
or a C₁-C₆ alkyl radical, optionally substituted with an -OH, -NHR₃, -SH, -COOH or -COOR₃ radical, in which R₃ represents a linear or branched C₁-C₄ alkyl radical,
or a C₇-C₁₂ aralkyl radical,
or a radical -CHR₄R₅ in which R₄ and R₅, which may be identical or different, represent a hydrogen atom or an optionally substituted phenyl radical or alternatively a 5- or 6-membered heterocycle;
the acylated forms thereof or alternatively the physiologically acceptable salts thereof, taken alone or as a mixture in any proportion;
for the preparation of a composition intended to treat seborrhoeic dermatitis and/or acne and/or blackheads and/or comedones.

4. Use according to any one of the preceding claims, **characterized in that** R₁ is a hydrogen atom or a methyl or ethyl radical.

5. Use according to the preceding claim, **characterized in that** R₁ is a hydrogen atom.

6. Use according to any one of the preceding claims, **characterized in that** R₂ is a hydrogen atom or a methyl radical.

7. Use according to the preceding claim, **characterized in that** R₂ is a hydrogen atom.

8. Use according to any one of the preceding claims, **characterized in, that** the compound is chosen from
4,6-dimethoxyindole-2-carboxylic acid
methyl 4,6-dimethoxyindole-2-carboxylate
N-methyl-4,6-dimethoxyindole-2-carboxylic acid
methyl N-methyl-4,6-dimethoxyindole-2-carboxylate
N-ethyl-4,6-dimethoxyindole-2-carboxylic acid.

9. Use according to the preceding claim, **characterized in that** the compound is 4,6-dimethoxyindole-2-carboxylic acid.

10. Use according to any one of the preceding claims, **characterized in that** the compound present is used in a concentration of between 0.001% and 20% by weight relative to the total weight of the composition.

11. Use according to any one of the preceding claims, **characterized in that** the compound present is used in a concentration of between 0.1% and 5% by .weight relative to the total weight of the composition.

12. Cosmetic process for treating greasy skin and/or a greasy scalp, this process consisting in applying to the skin and/or the scalp a composition comprising an effective amount of at least one compound corresponding to the general formula (I) as defined in any one of Claims 1 to 11, in leaving this composition in contact with the skin and/or the scalp, and in optionally rinsing it off.

## Patentansprüche

1. Nicht therapeutische, kosmetische Verwendung mindestens einer Verbindung der allgemeinen Formel (I) in einer wirksamen Menge in einer Zusammensetzung: 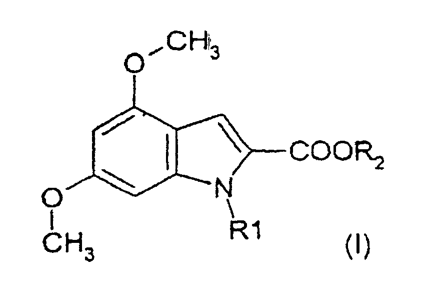 worin R₁ und R₂, die gleich oder verschieden sind, bedeuten:
. ein Wasserstoffatom,
. eine C₁₋₆-Alkylgruppe, die gegebenenfalls mit -OH, -NHR₃, -SH, -COOH oder -COOR₃ substituiert ist, wobei R₃ eine geradkettige oder verzweigte C₁₋₄-Alkylgruppe bedeutet,
. eine C₇₋₁₂-Aralkylgruppe,
. eine Gruppe -CHR₄R₅, wobei R₄ und R₅, die gleich oder verschieden sind, ein Wasserstoffatom, eine gegebenenfalls substituierte Phenylgruppe oder einen 5- oder 6-gliedrigen Heterocyclus bedeuten;
der acylierten Formen dieser Verbindungen oder physiologisch akzeptablen Salzen oder deren Gemischen in beliebigen Mengenanteilen;
als Wirkstoff zur Bekämpfung fettiger Haut und/oder fettiger Kopfhaut.

2. Verwendung mindestens einer Verbindung der allgemeinen Formel (I) in einer wirksamen Menge: 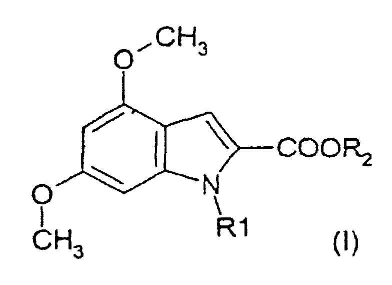 worin R₁ und R₂, die gleich oder verschieden sind, bedeuten:
. ein Wasserstoffatom,
. eine C₁₋₆-Alkylgruppe, die gegebenenfalls mit -OH, -NHR₃, -SH, -COOH oder -COOR₃ substituiert ist, wobei R₃ eine geradkettige oder verzweigte C₁₋₄-Alkylgruppe bedeutet,
. eine C₇₋₁₂-Aralkylgruppe,
. eine Gruppe -CHR₄R₅, wobei R₄ und R₅, die gleich oder verschieden sind, ein Wasserstoffatom, eine gegebenenfalls substituierte Phenylgruppe oder einen 5- oder 6-gliedrigen Heterocyclus bedeuten;
der acylierten Formen dieser Verbindungen oder physiologisch akzeptablen Salzen oder deren Gemischen in beliebigen Mengenanteilen;
zur Herstellung einer Zusammensetzung, die zur Behandlung von mit Seborrhoe zusammenhängenden Dermatosen vorgesehen ist.

3. Verwendung mindestens einer Verbindung der allgemeinen Formel (I) in einer wirksamen Menge: 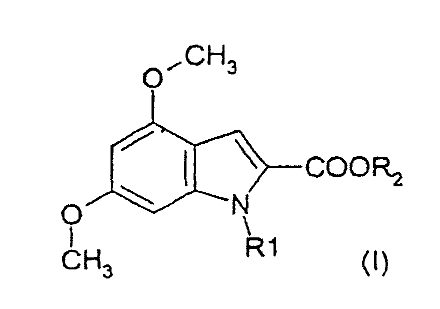 worin R₁ und R₂, die gleich oder verschieden sind, bedeuten:
. ein Wasserstoffatom,
. eine C₁₋₆-Alkylgruppe, die gegebenenfalls mit -OH, -NHR₃, -SH, -COOH oder -COOR₃ substituiert ist, wobei R₃ eine geradkettige oder verzweigte C₁₋₄-Alkylgruppe bedeutet,
. eine C₇₋₁₂-Aralkylgruppe,
. eine Gruppe -CHR₄R₅, wobei R₄ und R₅, die gleich oder verschieden sind, ein Wasserstoffatom, eine gegebenenfalls substituierte Phenylgruppe oder einen 5- oder 6-gliedrigen Heterocyclus bedeuten;
der acylierten Formen dieser Verbindungen oder physiologisch akzeptablen Salzen oder deren Gemischen in beliebigen Mengenanteilen;
zur Herstellung einer Zusammensetzung, die zur Behandlung von seborrhoeischer Dermatitis und/oder Akne und/oder Mitessern und/oder Komedonen vorgesehen ist.

4. Verwendung nach einem der vorhergehenden Ansprüche, **dadurch gekennzeichnet, dass** die Gruppe R₁ ein Wasserstoffatom, eine Methylgruppe oder eine Ethylgruppe bedeutet.

5. Verwendung nach dem vorhergehenden Anspruch, **dadurch gekennzeichnet, dass** R₁ ein Wasserstoffatom bedeutet.

6. Verwendung nach einem der vorhergehenden Ansprüche, **dadurch gekennzeichnet, dass** die Gruppe R₂ ein Wasserstoffatom oder eine Methylgruppe bedeutet.

7. Verwendung nach dem vorhergehenden Anspruch, **dadurch gekennzeichnet, dass** R₂ ein Wasserstoffatom bedeutet.

8. Verwendung nach einem der vorhergehenden Ansprüche, **dadurch gekennzeichnet, dass** die Verbindung ausgewählt ist unter:
4,6-Dimethoxy-indol-2-carbonsäure,
Methyl-4,6-dimethoxy-indol-2-carboxylat,
N-Methyl-4,6-dimethoxy-indol-2-carbonsäure,
N-Methyl-4,6-dimethoxy-indol-2-carbonsäuremethylester, und
N-Ethyl-4,6-dimethoxy-indol-2-carbonsäure.

9. Verwendung nach dem vorhergehenden Anspruch, **dadurch gekennzeichnet, dass** die Verbindung die 4,6-Dimethoxy-indol-2-carbonsäure ist.

10. Verwendung nach einem der vorhergehenden Ansprüche, **dadurch gekennzeichnet, dass** die Verbindung in einer Konzentration von 0,001 bis 20 Gew.-%, bezogen auf das Gesamtgewicht der Zusammensetzung, enthalten ist.

11. Verwendung nach einem der vorhergehenden Ansprüche, **dadurch gekennzeichnet, dass** die Verbindung in einer Konzentration von 0,1 bis 5 Gew.-%, bezogen auf das Gesamtgewicht der Zusammensetzung, enthalten ist.

12. Verfahren zur kosmetischen Behandlung von fettiger Haut und/oder fettiger Kopfhaut, das das darin besteht, eine Zusammensetzung, die eine wirksame Menge mindestens einer in einem der Ansprüche 1 bis 11 definierten Verbindung der allgemeinen Formel (I) enthält, auf die Haut und/oder die Kopfhaut aufzutragen, diese in Kontakt mit der Haut und/oder der Kopfhaut zu belassen und gegebenenfalls zu spülen.
